# EUROPEAN PATENT SPECIFICATION

(11) **EP 2 696 704 B1**
(45) Date of publication and mention of the grant of the patent: **22.09.2021**
(21) Application number: 12712103.6
(22) Date of filing: 28.03.2012
(51) Int. Cl.: A23L 2/00, A23L 2/08, A23L 2/10, A23L 2/38, A61K 31/00, A61K 31/715

(54) **PROCESS FOR THE MANUFACTURE OF A POWDER CONTAINING LUTEIN**
VERFAHREN ZUR HERSTELLUNG VON LUTEINHALTIGEM PULVER
PROCÉDÉ POUR LA FABRICATION D'UNE POUDRE CONTENANT DE LA LUTÉINE

(30) Priority: 13.04.2011 CH 669112011
(43) Date of publication of application: 19.02.2014
(73) Proprietor: DSM IP Assets B.V., 6411 TE Heerlen (NL)
(72) Inventor: SCHAEFER, Christian, CH-4303 Kaiseraugst (CH); SCHLEGEL, Bernd, CH-4334 Sisseln (CH)
(74) Representative: Kurt, Manfred
(86) International application number: PCT/EP2012/055550
(87) International publication number: WO 2012/139895

(56) References cited:
- WO-A1-2007/090614
- DE-A1-102004 046 026
- DATABASE WPI Week 201062 Thomson Scientific, London, GB; AN 2010-L42653 XP002674981, & CN 101 803 769 A (ZHU J) 18 August 2010 (2010-08-18)
- None

## Description

The present invention relates to a dry, powder instant beverage product that is obtainable by a process for the manufacture of a powder containing lutein.

Lutein, which is an important member of the carotenoid family, is a useful coloring agent for a variety of foods and beverages. Lutein also has further beneficial properties in addition to its coloring capacity. For this reason, lutein is often included in foods and beverages as a colorant, especially where their further beneficial property, such as vitamin A fortification, is needed or desired. DE 102004046025 A1 discloses the preparation of carotenoid dry powders. WO 2007/090614 a process for the manufacture of an improved modified polysaccharide. CN101803769 A discloses a soup base, which is useful for human consumption and industrial productions.

Although in most applications both, the coloring effect and the further beneficial property, is desired, there are other applications where the intensive color of lutein is an issue with regard to the desired color of the end product, especially when the other properties of the lutein are in the focus of the application.

It was therefore an object of the following invention to provide a powder containing lutein wherein the powder in a preferred case should have only minor coloring effects on the end product. Furthermore the powder should satisfy the usual demands of a food composition, such as being stable against oxidation, being and staying evenly distributed in the product over time and so on.

Furthermore the goal was to produce an instant beverage powder, which is easy to produce and which has improved properties with regard to fast solubility (also with cold solvents, especially cold water).

It has surprisingly been found that the object of the present invention is achieved by a dry, powder instant beverage product which before its consumption is to be mixed with water or another liquid beverage composition, said instant beverage containing a powder containing lutein obtained by a process for the manufacture of a powder containing lutein comprising the steps of
a) providing an aqueous solution / suspension of a polysaccharide;
b) forming a suspension of lutein in the solution / suspension of step a);
c) milling the suspension of step b);
d) drying the suspension of step c),
characterized in that the mean particle size of the lutein particles in step c) is in a range of from 50 nm to 500 nm.

The present invention is defined by the appended claims. It was not to be foreseen by the person skilled in the art that a powder obtainable by the process according to the present invention would solve the above mentioned issues.

Step a) of the process according to the present invention can be conducted at any reasonable temperature to ensure a rapid dissolution of the polysaccharide in water. To ensure complete dissolution of the polysaccharide within a reasonable amount of time, heating to about 40 to 80 °C is preferable.

The term "polysaccharide" as used herein includes acacia gum, pectins, celluloses, cellulose derivatives, and/or modified polysaccharides.

The term "modified polysaccharide" as used herein relates to polysaccharides which contain a lipophilic moiety, e. g. a hydrocarbon moiety having a chain length of preferably 5 to 18 carbon atoms in the straight chain. Preferably the modified polysaccharide should be acceptable for human consumption, i.e. preferred modified polysaccharides should be GRAS (generally recognized as safe) or approved for food consumption as determined by the various regulatory agencies world wide. Preferred modified polysaccharides are glucose (syrup) and modified food starch.

The term "modified food starch" as used herein relates to modified starches that are made from starches substituted by known chemical methods with hydrophobic moieties. For example starch may be treated with cyclic dicarboxylic acid anhydrides such as succinic and/or glutaric anhydrides, substituted with an alkyl or alkenyl hydrocarbon group.

A particularly preferred modified starch of this invention has the following formula (I) 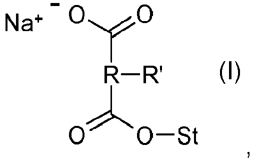 wherein St is a starch, R is an alkylene radical and R' is a hydrophobic group. Preferably R is a lower alkylene radical such as dimethylene or trimethylene. R' may be an alkyl or alkenyl group, preferably having 5 to 18 carbon atoms. A preferred modified starch of formula (I) is starch sodium octenyl succinate ("OSA-starch"). The term "OSA-starch" as used herein denotes any starch (from any natural source such as corn, wheat, tapioca, potatoe or synthesized) that was treated with octenyl succinic anhydride (OSA). The degree of substitution, i.e. the number of esterified hydroxyl groups with regard to the total number of hydroxyl groups usually varies in a range of from 0.1 to 10 %, preferably in a range of from 0.5 to 5 %, more preferably in a range of from 2 to 4 %.

OSA-starches may contain further hydrocolloids, such as starch, maltodextrin, carbohydrates, gum, corn syrup, etc. and optionally any typical emulsifier (as co-emulgator), such as mono- and diglycerides of fatty acids, polyglycerol esters of fatty acids, lecithins, sorbitan monostearate, and plant fibre or sugar.

OSA-starches are commercially available e.g. from National Starch under the trade names HiCap 100, Capsul, Capsul HS, Purity Gum 2000, Purity Gum Ultra, UNI-PURE, HYLON VII; from Roquette Frères; from Cerestar under the trade name C*EmCap or from Tate & Lyle.

It is advantageous if the quantities of water and polysaccharide(s) (one or more compounds) in the aqueous solution of step a) are selected so that the amount of polysaccharide(s) in the suspension according to step b) of the present invention is in the range of from 10 to 25 % by weight, and the amount of water in said suspension is in the range of from 50 to 70 % by weight, preferably from 55 to 65 % by weight, each based on the total weight of the suspension.

According to the present invention it is advantageous if the amount of lutein added in step b) is in the range of from 2 to 11 % by weight, preferably from 5 to 10 % by weight, each based on the total weight of the suspension.

In a preferred embodiment of the process of the present invention further adjuvants are added to the solution of step a) in step b). The further adjuvants are preferably selected from one or more of the following groups:
- diluents;
- antioxidants (fat-soluble or water-soluble);
- triglycerides (oils and/or fats).

Preferred diluents can be selected from glycerol, mono-, di- and oligosaccharides. According to the present invention sucrose, invert sugar, glucose, fructose, lactose, maltose, saccharose, sugar alcohols and starch hydrolysates, such as dextrins and maltodextrins are preferred. Maltodextrins are especially preferred.

According to the present invention it is advantageous if the amount of diluents (one or more compounds) in the suspension is in the range of from 5 to 20 % by weight, preferably from 10 to 15 % % by weight, each based on the total weight of the suspension.

Preferred water-soluble antioxidants are for example ascorbic acid or salts thereof, preferably sodium ascorbate. Preferred fat-soluble antioxidants are for example tocopherol (synthetic or natural); butylated hydroxytoluene (BHT); butylated hydroxyanisole (BHA); propyl gallate; tert. butyl hydroxyquinoline and/or ascorbic acid esters of a fatty acid, preferably ascorbyl palmitat and/or ascorbyl stearate. dl-Tocopherol is especially preferred.

According to the present invention it is advantageous if the amount of antioxidant(s) (one or more compounds) in the suspension is in the range of from 0.1 to 2 % by weight, preferably from 0.5 to 1.5 % by weight, each based on the total weight of the suspension.

The triglyceride is preferably selected from vegetable oils and/or fats, preferably corn oil, sunflower oil, soybean oil, safflower oil, rape seed oil, peanut oil, palm oil, palm kernel oil, cotton seed oil and/or coconut oil, including fractionated qualities thereof. The triglycerides can preferably be so-called MCT (medium chain triglycerides), i.e. ester of medium chain fatty acids (preferably saturated fatty acids with a chain length of 6 to 12 C atoms) and glycerol.

According to the present invention it is advantageous if the amount of triglyceride(s) (one or more compounds) in the suspension is in the range of from 0 to 5 % by weight, preferably from 0.2 to 2 % by weight, each based on the total weight of the suspension.

The milling step is preferably carried out with a commercially available ball mill. The desired mean particle size of the lutein particles is achieved by adjusting the following parameters with respect to each other: rotor speed (peripheral speed), mean residence time in the mill, material and size of the milling beads and load of the mill. Preferred is a low rotor speed (e.g. peripheral speeds of from 1 to 5 m/s, preferably from 2 to 4 m/s), a short mean residence time in the mill (e.g. 1 to 10 min), glass beads (e.g. with a mean diameter of 0.5 to 1.5 mm) and an average load of 70 to 90 %.

The preferred milling parameters may differ depending on the ball mill used in the milling step, but can easily be adjusted by the person skilled in the art through no inventive fault of his own.

Step c) of the process according to the present invention can be conducted at any reasonable temperature. Heating to about 40 to 60 °C is preferable.

The drying step may be carried out with any conventional drying process known to the person skilled in the art, preferred are spray drying and/or a powder catch process where sprayed suspension droplets are caught in a bed of an adsorbant such as starch or calcium silicate or silicic acid or calcium carbonate or mixtures thereof and subsequently dried.

According to the present invention it is advantageous if the residual moisture content in the powder obtained by the drying step is in the range of from 4 to 6 weight-%, based on the total weight of the powder.

It is also disclosed that the suspension of step c) is spray-dried. In this case it is preferred to select the spray drying parameters as follows:
- Air inlet: about 220 to 160 °C, especially about 180 °C;
- Air exit: about 100 to 60 °C, especially about 80 °C.

It is also disclosed that one or more flow-conditioning agents (also referred to as anti-caking agents, flow enhancer) are added to the powder, i.e. during the drying step or to the product that is obtained in step d).

Preferred flow-conditioning agents are for example (hydrophilic) fumed silica, such as those commercially available under the trade name AEROSIL® from Degussa.

According to the present invention it is advantageous if the amount of flow-conditioning agent(s) (one or more compounds) in the powder is in the range of from 0.1 to 0.5 % by weight, based on the total weight of the powder.

The present invention is directed to the powder (dry powder) obtained by the process of the present invention as disclosed above.

There is also the disclosure of a food composition, especially to a beverage containing the powder obtained/obtainable by the described process. The beverage of the present invention may be a base composition to which upon its use water or another liquid beverage composition (such as milk, juice and so on) can or has to be added. The base composition can be prepared as a dry, powder product (instant beverage) which before its consumption is to be mixed with water or another liquid beverage composition, as a concentrate to which water or another liquid beverage composition has to be added, or as a beverage to which no liquid needs to be added.

The amount of powder according to the invention which is to be added to a food composition depends on the potency of said powder, i.e. the amount of lutein in the powder, which according to the present invention can range from about 1 to about 20 % by weight, preferably from about 1 to about 10 % by weight, each based on the total weight of the powder.

In the case of clear beverages, the preferred beverage should have an optical clarity which does not differ significantly from its optical clarity before addition of the powder, for example which does not appear significantly more turbid on visual inspection.

It was not to be foreseen by the person skilled in the art that addition of the powder according to the present invention to water or another liquid beverage composition (such as juice and so on) would not change the color of the liquid, i.e. would not have a coloring effect visible to the naked eye on the beverage.

Effervescent tablets comprising the powder of this invention are also part of this invention. The tablets of this invention may also be dissolved in a liquid without changing the color with regard to a similar effervescent tablet not containing the powder according to the present invention.

The invention is further illustrated by the following examples.

### Example

312 g OSA-starch (E1450) and 41.2 g maltodextrin were added to 364 g of destilled water at 25 °C and then stirred and heated to 45 °C until the mixture was homogenous. Afterward the mixture has been cooled down and 10.8 g sodium ascorbate have been added and the pH of the mixture has been adjusted to 3.5 by H₂SO₄.

24.3 g Lutein and 3.6 g dl-α-tocopherol were added to this mixture. The mixture was stirred and then wet milling was carried out by usage of a DISPERMAT® SL (from VMA-GETZMANN, Germany) and zirconia beads, diameter 0.4 mm (from Sigmund Lindner, Germany), to a mean particle diameter size of about 0.38 µm as determined via photon correlation spectroscopy (PCS). The milled suspension was then spray dried.

The so obtained powder had good dissolution properties.

## Claims

1. Dry, powder instant beverage product which before its consumption is to be mixed with water or another liquid beverage composition said instant beverage containing a powder containing lutein obtained by the following process comprising the steps of
a) providing an aqueous solution/suspension of a polysaccharide;
b) forming a suspension of lutein in the solution/suspension of step a);
c) milling the suspension of step b);
d) drying the suspension of step c),
**characterized in that** the mean particle size of the lutein particles after the milling in step c) is in a range from 50 nm to 500 nm.

2. The dry, powder product according to claim 1, wherein the process is **characterized in that** the polysaccharide is selected from acacia gum, pectins, celluloses, cellulose derivatives and/or polysaccharides.

3. The dry, powder product according to any of the preceding claims, wherein the process is **characterized in that** the polysaccharide is selected from modified polysaccharides.

4. The dry, powder product according to any of the preceding claims, wherein the process is **characterized in that** the quantities of water and polysaccharide(s) (one or more compounds) in the aqueous solution of step a) are selected so that the amount of polysaccharide(s) in the suspension according to step b) of the present invention is in the range of from 1 to 25 % by weight, and the amount of water in said suspension is in the range of from 50 to 70 % by weight, each based on the total weight of the suspension.

5. The dry, powder product according to any of the preceding claims, wherein the process is **characterized in that** the amount of lutein added in step b) is in the range of from 2 to 11 % by weight, based on the total weight of the suspension.

6. The dry, powder product according to any of the preceding claims, wherein the process is **characterized in that** in step b) further adjuvants selected from one or more of the following groups of diluents; fat-soluble or water-soluble antioxidants; triglycerides such as oils and/or fats, are added to the solution of step a).

7. The dry, powder product according to any of the preceding claims, wherein the process is **characterized in that** the milling step is carried out with a ball mill.

8. The dry, powder product according to any of the preceding claims, wherein the process is **characterized in that** the drying step is a spray drying step.

## Patentansprüche

1. Trockenes, pulverförmiges Instantgetränkeprodukt, das vor seinem Verbrauch mit Wasser oder einer anderen flüssigen Getränkezusammensetzung zu mischen ist, wobei das Instantgetränk ein Lutein-enthaltendes Pulver enthält, das durch das folgende Verfahren erhalten ist, das die Schritte umfasst:
a) Bereitstellen einer wässrigen Lösung/Suspension eines Polysaccharids;
b) Bilden einer Suspension von Lutein in der Lösung/Suspension von Schritt a);
c) Mahlen der Suspension von Schritt b);
d) Trocknen der Suspension von Schritt c),
**dadurch gekennzeichnet, dass** die mittlere Partikelgröße der Luteinpartikel nach dem Mahlen bei Schritt c) in dem Bereich von 50 nm bis 500 nm liegt.

2. Trockenes Pulverprodukt gemäß Anspruch 1, wobei das Verfahren **dadurch gekennzeichnet ist, dass** das Polysaccharid aus Gummi arabicum, Pektinen, Cellulosen, Cellulosederivaten und/oder Polysacchariden ausgewählt ist.

3. Trockenes Pulverprodukt gemäß einem der vorstehenden Ansprüche, wobei das Verfahren **dadurch gekennzeichnet ist, dass** das Polysaccharid aus modifizierten Polysacchariden ausgewählt ist.

4. Trockenes Pulverprodukt gemäß einem der vorstehenden Ansprüche, wobei das Verfahren **dadurch gekennzeichnet ist, dass** die Mengen von Wasser und Polysaccharid(en) (eine oder mehrere Verbindungen) in der wässrigen Lösung von Schritt a) so ausgewählt sind, dass die Menge von Polysaccharid(en) in der Suspension gemäß Schritt b) der vorliegenden Erfindung in dem Bereich von 1 bis 25 Gew.-% liegt und die Menge von Wasser in der Suspension in dem Bereich von 50 bis 70 Gew.-% liegt, jeweils bezogen auf das Gesamtgewicht der Suspension.

5. Trockenes Pulverprodukt gemäß einem der vorstehenden Ansprüche, wobei das Verfahren **dadurch gekennzeichnet ist, dass** die Menge von bei Schritt b) zugegebenem Lutein in dem Bereich von 2 bis 11 Gew.-%, bezogen auf das Gesamtgewicht der Suspension, liegt.

6. Trockenes Pulverprodukt gemäß einem der vorstehenden Ansprüche, wobei das Verfahren **dadurch gekennzeichnet ist, dass** bei Schritt b) ferner Hilfsstoffe ausgewählt aus einem oder mehreren der folgenden Gruppe von Verdünnungsmitteln; fettlöslichen oder wasserlöslichen Antioxidationsmitteln; Triglyceriden, wie z. B. Ölen und/oder Fetten, zu der Lösung von Schritt a) zugegeben werden.

7. Trockenes Pulverprodukt gemäß einem der vorstehenden Ansprüche, wobei das Verfahren **dadurch gekennzeichnet ist, dass** der Mahlschritt in einer Kugelmühle durchgeführt wird.

8. Trockenes Pulverprodukt gemäß einem der vorstehenden Ansprüche, wobei das Verfahren **dadurch gekennzeichnet ist, dass** der Trocknungsschritt ein Sprühtrocknungsschritt ist.

## Revendications

1. Produit de boisson instantané sec en poudre, qui, avant sa consommation, doit être mélangé avec de l'eau ou une autre composition de boisson liquide, ladite boisson instantanée contenant une poudre contenant de la lutéine obtenue par le procédé suivant, comprenant les étapes consistant à :
a) mettre à disposition une solution/suspension aqueuse d'un polysaccharide ;
b) former une suspension de lutéine dans la solution/suspension de l'étape a) ;
c) broyer la suspension de l'étape b) ;
d) sécher la suspension de l'étape c) ;
**caractérisé en ce que** la taille de particules moyenne des particules de lutéine après le broyage dans l'étape c) se trouve dans la plage allant de 50 nm à 500 nm.

2. Produit sec en poudre selon la revendication 1, le procédé étant **caractérisé en ce que** le polysaccharide est choisi parmi la gomme arabique, les pectines, les celluloses, les dérivés de cellulose, et/ou les polysaccharides.

3. Produit sec en poudre selon l'une quelconque des revendications précédentes, le procédé étant **caractérisé en ce que** le polysaccharide est choisi parmi les polysaccharides modifiés.

4. Produit sec en poudre selon l'une quelconque des revendications précédentes, le procédé étant **caractérisé en ce que** les quantités d'eau et de polysaccharide(s) (un ou plusieurs composés) dans la solution aqueuse de l'étape a) sont choisies de façon à ce que la quantité de polysaccharide(s) dans la suspension selon l'étape b) de la présente invention se trouve dans la plage allant de 1 à 25% en poids,
et que la quantité d'eau dans ladite suspension se trouve dans la plage allant de 50 à 70% en poids, chacune sur la base du poids total de la suspension.

5. Produit sec en poudre selon l'une quelconque des revendications précédentes, le procédé étant **caractérisé en ce que** la quantité de lutéine ajoutée dans l'étape b) se trouve dans la plage allant de 2 à 11% en poids, sur la base du poids total de la suspension.

6. Produit sec en poudre selon l'une quelconque des revendications précédentes, le procédé étant **caractérisé en ce que**, dans l'étape b), des adjuvants supplémentaires choisis parmi un ou plusieurs parmi les groupes suivants de diluants ; d'antioxydants liposolubles ou hydrosolubles ; de triglycérides tels que les huiles et/ou les matières grasses ; sont ajoutés à la solution de l'étape a) .

7. Produit sec en poudre selon l'une quelconque des revendications précédentes, le procédé étant **caractérisé en ce que** l'étape de broyage est effectuée dans un broyeur à boulets.

8. Produit sec en poudre selon l'une quelconque des revendications précédentes, le procédé étant **caractérisé en ce que** l'étape de séchage est une étape de séchage par pulvérisation.
